# EUROPEAN PATENT APPLICATION

(11) **EP 2 223 705 A1**
(43) Date of publication of application: **01.09.2010**
(21) Application number: 09075064.7
(22) Date of filing: 09.02.2009
(51) Int. Cl.: A61L 9/12

(54) **Volatiles delivering device applicable to forced ventilation apparatuses**

(71) Applicant: Volonté, Maurizio, 21042 Caronno Pertusella (VA) (IT); Volonté, Massimo, 21042 Caronno Pertusella (VA) (IT); Ferrari, Davide, 21040 Cislago (VA) (IT)
(72) Inventor: Volonté, Maurizio, 21042 Caronno Pertusella (VA) (IT); Volonté, Massimo, 21042 Caronno Pertusella (VA) (IT); Ferrari, Davide, 21040 Cislago (VA) (IT)
(74) Representative: Riccardi, Sergio

(57) **Abstract**

There is described a device delivering volatiles applicable to forced ventilation apparatuses at the intake side thereof, characterized by the fact that it is constituted by an envelope comprising a bottom portion or base and a top portion or cover both provided with grids or holes for passing of air through the envelop interior, wherein a solid material soaked in flavouring, deodorizing and/or purifying volatile is housed.

## Description

The present invention relates to a device adapted to deliver volatiles for flavouring, deodorizing, purifying and sanitizing rooms, applicable onto the intake side of any forced ventilation apparatus, such as conditioners, automatic conditioners, convector, ventilators and combinations thereof or like apparatuses.

A device of the sort is known for example from the US 6,749,672 B2 and it is constituted by a frame applicable to a ventilation duct, housing a cartridge soaked in scent or deodorant. However, such cartridge has to be periodically replaced once the volatile is exhausted and therefore the device has to be taken apart and opened for the recharge. Moreover, the frame or envelop of the device obstructs the air flow into the ventilation duct, decreasing or at least altering the air flow generated into the ventilation duct or apparatus.

The device according to the present invention has been designed in order to overcome a drawback existing in all the interiors, that is the flavouring and deodorization of the interior naturally and without any direct power consumption, by using all the sources regarding the forced ventilation, since applied on the intake side of any apparatus of the sort.

The device is easily applicable, suitable for all the air conditioners, convectors, ventilators and all the forced ventilation apparatuses, and by a small hooking allows in a really simple manner the positioning and removal from any forced ventilation conditioner, ventilator, convector.

Such hooking occurs by means of one or two tangs in plastic material placed onto the bottom of the device, which has to be positioned on the top of the conditioner, next to the air intake grid which usually rests on the top of the inner unit of the subject-matter apparatus.

The device is easily used in all the under mentioned environments, such as residential structures, shopping centres and every public spaces and where there is the presence of a forced ventilation apparatus: conditioner, ventilator, convector, pellet stoves, hairdryers, automatic conditioners and so on.

The advantaged offered by this new deoconditioner is to spread pleasant scent healthy in the environment through the air by a release gradual and adjustable directly from the one's own air conditioning system or ventilation apparatus used. The device by its flavouring removes unpleasant odours caused by the condensate release within the conditioner, and also serves as insecticide in the environment.

Unlike others environment perfumings the perfuming substance is in its solid state and not in liquid state, therefore assure a greater efficiency during time.

The device is disposable, therefore does not provide any type of recharge, and it is really easily applicable outside any apparatus, unlike some others perfumings not requiring installation.

It does not decrease nor alters the air flow generated by the ventilation apparatus but it indirectly undergoes the intensity of the air flow produced by the apparatus it is applied to. Furthermore, a sliding aperture could be provided in order to adjust the intensity of the flavouring.

Once placed onto an air conditioning apparatus it does not decrease nor alters the cooling efficiency (cooling power) the system spreads in the air nor decreases nor alters the hot efficiency (heat power) the system spreads in the air.

Unlike others perfumings the device does not need a determined temperature allowing the flavour for spreading in the air.

The product is able to spread flavouring through: cold air, hot air, dehumidified air, humidified air.

The device has an design and ergonomic and technical shape studied in order to use intake and circulation of the air entering the ventilation apparatus.

A preferred embodiment of the device according to the present invention is now described in detail, as only explanatory example not limiting its scope, with reference to the annexed illustrative drawings, wherein:

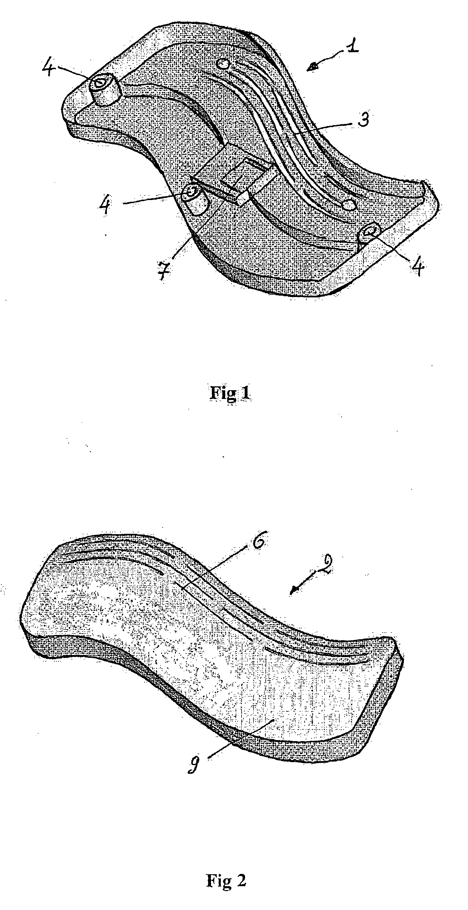

fig. 1 is a view of the inner side of the bottom or envelope base;
fig. 2 is a view of the outer side of the top or cover of the same envelope;

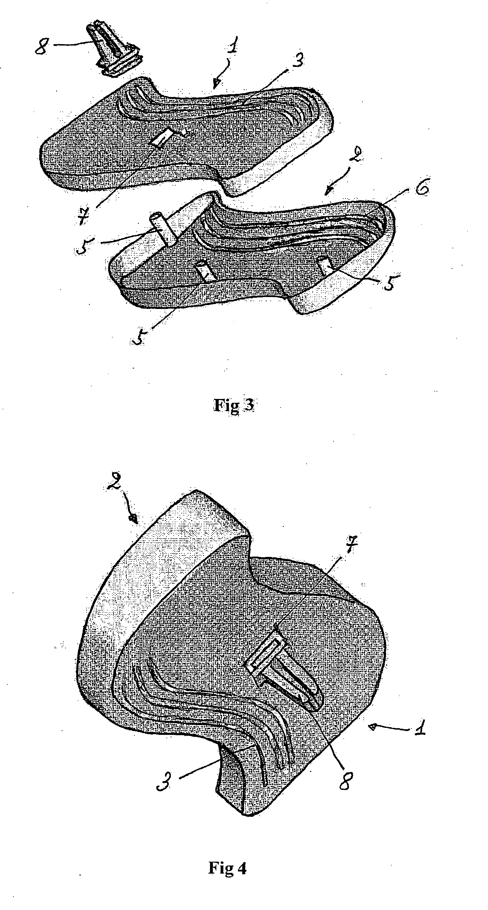

fig. 3 is an exploded view of the two parts before their assembly;
fig. 4 is a perspective view of the assembled device.

Referring now to the figures of the annexed drawings, the delivering device according to the invention, in its preferred embodiment, is constituted by an envelope in plastic material, having a parallelepiped shape with preferably wavy sides, comprising a bottom portion or base 1 and a top portion or cover 2.

The bottom portion or base 1 has grids or air holes 3, slots 4 for pins 5 for assembly together with the top portion or cover 2 even provided with grids or holes 6 for the air intake. In the bottom portion or base 1 a slide guide 7 is formed for a tang or tweezers 8 hooking the ventilation apparatus. On the outer face of the top portion or cover, besides the inscription of the product name there are marked symbols 9 corresponding the four operating modes: cold, hot, ventilation, dehumidification.

In the bottom portion or base 1 the material emitting volatiles is housed, which can be a web in EVA (vinyl acetate ethylene) in micro-pierced mould or granules or even wood balls or other porous material. In any case the recharge substance does not contain alcohols or other solvents and uses a wide flavouring range. The device is disposable, therefore once exhausted the recharge it must be thrown and replaced, and could be manufactured with multiple and various shapes and multiple colorations.

As just mentioned, a preferred embodiment has been previously described but it has to be noticed that numerous modifications, adds and/or replacements could be brought thereto without falling for this reason outside the its scope, as it ensues even from the annexed claims.

## Claims

1. A volatiles delivering device applicable to the forced ventilation apparatuses onto their intake side, **characterized by the fact that** it is constituted by an envelope comprising a bottom portion or base for a top portion or cover both provided with grids or holes for passing of the air through the envelope interior, wherein a solid material soaked in volatiles flavouring, deodorizing and/or purifying.

2. The delivering device according to claim 1, **characterized by the fact that** the bottom portion or base has slots for insertion of pins formed from the top portion or cover for assembling the two portions constituting a closed disposable envelope.

3. The delivering device according to claim 1, **characterized by the fact that** the bottom portion or base is provided with a guide for a tang or tweezers hooking ventilation apparatus.

4. The delivering device according to claim **characterized by the fact that** in the outer face of the top portion or cover symbols corresponding to the four operating modes such as cold, hot, ventilation, dehumidification are marked.

5. The delivering device according to one or more of the preceding claims, **characterized by the fact that** the recharge material is constituted by a web made of EVA (vinyl acetate ethylene), granules, wood balls or other porous material.

6. The delivering device according to one or more of the preceding claims, **characterized by the fact that** the envelope is formed with plastic material.

7. The delivering device according to one or more of the preceding claims, **characterized by the fact that** the recharge material is devoid of alcohols, solvents or other substances polluting for the environment.

8. The delivering device according to one or more of the preceding claims, **characterized by the fact that** the recharge material also comprises an insecticide substance.
